# EUROPEAN PATENT APPLICATION

(11) **EP 1 764 417 A1**
(43) Date of publication of application: **21.03.2007**
(21) Application number: 06120764.3
(22) Date of filing: 15.09.2006
(51) Int. Cl.: C12Q 1/18

(54) **Method and apparatus for electrically detecting an adverse effect of a toxic substance on procaryotic cells**

(30) Priority: 15.09.2005 KR 20050086420
(71) Applicant: Samsung Electronics Co., Ltd., Suwon-si, Gyeonggi-Do (KR)
(72) Inventor: Choi, Soo Hyung, 215-502 Shinyoungtong 2nd Apt, Hwaseong-si, Gyeonggi-do 445-983 (KR); Han, Jung Im, Gwanag-gu, Seoul 151-847 (KR); Cho, Yoon Kyoung, 102-1702 Dongsuwon LG Village, Suwon-si, Gyeonggi-do 443-706 (KR)
(74) Representative: Grünecker, Kinkeldey, Stockmair & Schwanhäusser Anwaltssozietät

(57) **Abstract**

A method and apparatus for electrically measuring an adverse effect of toxic substances on prokaryotic cells includes measuring a whole intracellular change caused by the presence of toxic substances as a change in an electric signal of cell membranes of the prokaryotic cells. According to the present invention, it is possible to electrically measure the presence of toxicity and the extent of the adverse effect very easily and within a rapid period of time and also to measure the adverse effect of toxic substances regardless of their type. Thus, the method and apparatus of the present invention have wide applicability and can be easily applied to a lab-on-a-chip.

## Description

This application claims priority to Korean Patent Application No. 10-2005-0086420, filed September 15, 2005, and all the benefits accruing therefrom under 35 U.S.C. § 119, the contents of which in its entirety are herein incorporated by reference.

### BACKGROUND OF THE INVENTION

### 1. Field Of The Invention

The present invention relates to a method and apparatus for electrically detecting an adverse effect of toxic substances on prokaryotic cells, and more particularly, to a method and apparatus for electrically detecting an adverse effect of toxic substances by measuring a whole intracellular change caused by the presence of the toxic substances as a change in an electric signal of cell membranes of the prokaryotic cells.

### 2. Description of the Related Art

The human body and environment have been threatened with various hazardous factors including the occurrence of damage due to the increase of new toxic substances as a result of industrialization (e.g., the threat of endocrine disruptors), ecosystem destruction owing to the increase of wastes or pesticide ingredients acting as a water pollutant, the severity of residual pesticide ingredients in foods, and heavy metal pollution in soil which increases day by day, for example, but is not limited thereto.

It has therefore been raised as a very important and imminent matter to monitor such chemicals that have a bad influence upon the human body and environment.

More than eighty thousand chemicals have been commercially used at home and abroad at present, and several thousands of new chemicals have been developed and used as raw materials for foods, medicines, cosmetics, medical instruments and the like year after year. Therefore, in order to study how harmful such chemicals are to the human body and environment, it is very important to precisely examine the influence of toxicity and the extent of exposure thereof.

There is an environment risk assessment as a part of the methods used for securing the safety of these chemicals, wherein legally acceptable guidelines of safety standards for environmental toxicity are prepared to analyze and evaluate the hazardous and adverse effects of these chemicals upon the human body and environment, directly and indirectly. Through such environment risk assessment, all information for a certain chemical compound, such as the amount of exposure, degradation rate, concentration rate, toxicity and so on, are analyzed and collected, thereby allowing a reasonable evaluation for that compound and deducing an appropriate management program based on such evaluation.

To assess such environment risk at an early stage and rapidly cope with the same, there is a need for a widespread environmental monitoring method. For example, several methods have been used for detecting an adverse effect with recombinant microorganisms, luminous bacteria, yeasts or enzymes. These methods mainly measure the adverse effect by an optical technique.

U.S. Patent No. 5,278,048 discloses a method of attaching prokaryotic or eukaryotic cells onto the surface of a silicon sensor in a micro chamber, pouring a cell affecting agent thereinto, and then measuring pH change of 0.1 to 0.5 units within 1 to 4 minutes. However, since this method measures the pH change on the silicon sensor only when an H⁺ concentration change of cells happens, it cannot detect other intracellular changes excluding the H⁺ concentration change, thereby making it impossible to exactly identify the presence of a cell affecting agent.

U.S. Patent Application Publication No. 2004/0152067 teaches a method for measuring intracellular toxicity through a change in impedance by employing an apparatus including a first chamber accommodating a cell suspension, a second chamber located at the lower side of the first chamber and having an electrode, and a porous membrane located between the first and the second chambers. This method employs mammalian cells having high adhesiveness to the electrode. However, the mammalian cells used in this method need to be cultured for a long time to satisfy strict prerequisites for cell stability, which is not suitable for use in a portable sensor for detecting toxicity and a sensing method therefor.

### BRIEF SUMMARY OF THE INVENTION

The inventors of the present invention have therefore endeavored to overcome these problems of the prior art, and found that a cell membrane of prokaryotic cells shows a different electric impedance signal according to the presence or absence of toxic substances, according to the present invention. As a result, the present invention provides a method and apparatus for electrically detecting an adverse effect of toxic substances by measuring a whole intracellular change caused by the presence of toxic substances as a change in an electric signal of cell membranes of prokaryotic cells within a rapid period of time. The method and apparatus of the present invention can be effectively applied to a toxicity detection sensor and is a suitable sensing method for a portable lab-on-α-chip.

Accordingly, a primary aspect or feature of the present invention provides a method and apparatus for electrically detecting an adverse effect of toxic substances by measuring a change in an electric signal of cell membranes of prokaryotic cells.

To accomplish the above aspect or feature of the present invention, a method for electrically detecting an adverse effect of toxic substances is provided as an exemplary embodiment of the present invention. The method includes: immobilizing prokaryotic cells on a surface of an electrode or a transistor; contacting the immobilized prokaryotic cells with a sample containing a toxic substance to be analyzed; and measuring an electric signal of cell membranes of prokaryotic cells.

In another exemplary embodiment, an apparatus for detecting an adverse effect of a toxic substance using prokaryotic cells is provided. The apparatus includes: a measuring part which detects a change in an electric signal of cell membranes of the prokaryotic cells; and the prokaryotic cells immobilized on the measuring part.

### BRIEF DESCRIPTION OF DRAWINGS

The above and other aspects, features and advantages of the present invention will become more apparent from the following description of exemplary embodiments taken in conjunction with the accompanying drawings, in which:
FIG 1 is a schematic diagram of an apparatus for measuring impedance using immobilized cells on a surface of an electrode;
FIGS. 2A to 2C are graphs comparing impedance measured after a cells-immobilized chip is treated with 20% ethanol as a toxic substance with impedances of two control groups (e.g., Controls 1 and 2);
FIG 3 is a graph showing a change in E' (dielectric permittivity) and E" (dielectric losses) signal patterns of a cell membrane that is disclosed in an article of a prior art;
FIGS. 4A to 4C are graphs showing the results of impedance measured after a cells-immobilized chip is treated with carbonyl cyanide m-chlorophenoxylhydrazone ("CCCP") as a toxic substance, and sorbitol and LB broth as a non-toxic substance, respectively;
FIG. 5A is a bar graph comparing the result of a colony counted when a cells-immobilized chip is treated with CCCP with that of an untreated control;
FIG. 5B is a bar graph comparing the result of optically measuring a change in cell membrane potential when a cells-immobilized chip is treated with CCCP with that of an untreated control; and
FIG 6 is a bar graph comparing the result of optically measuring a change in cell membrane potential when a cells-immobilized chip is treated with 20% ethanol with that of an untreated control.

### DETAILED DESCRIPTION OF THE INVENTION

The present invention now will be described more fully hereinafter with reference to the accompanying drawings, in which exemplary embodiments of the present invention are shown. The present invention may, however, be embodied in many different forms and should not be construed as limited to the exemplary embodiments set forth herein. Rather, these exemplary embodiments are provided so that this disclosure will be thorough and complete, and will fully convey the scope of the invention to those skilled in the art. Like reference numerals refer to like elements throughout.

It will be understood that when an element is referred to as being "on" another element, it can be directly on the other element or intervening elements may be present therebetween. In contrast, when an element is referred to as being "directly on" another element, there are no intervening elements present. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another elements as illustrated in the figures. It will be understood that relative terms are intended to encompass different orientations of the device in addition to the orientation depicted in the figures. For example, if the device in one of the figures is turned over, elements described as being on the "lower" side of other elements would then be oriented on "upper" sides of the other elements. The exemplary term "lower", can therefore, encompasses both an orientation of "lower" and "upper," depending of the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments of the present invention are described herein with reference to cross section illustrations that are schematic illustrations of idealized embodiments of the present invention. As such, variations from the shapes of the illustrations as a result, for example, of manufacturing techniques and/or tolerances, are to be expected. Thus, exemplary embodiments of the present invention should not be construed as limited to the particular shapes of regions illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present invention.

Hereinafter, the present invention will be described in more detail with reference to the accompanying drawings.

In an exemplary embodiment, the present invention provides a method for electrically detecting an adverse effect of a toxic substance, which includes: immobilizing prokaryotic cells on a surface of an electrode or a transistor; contacting the immobilized prokaryotic cells with a sample containing the toxic substance to be analyzed; and measuring an electric signal of cell membranes of the prokaryotic cells.

In the method of the present invention, the immobilizing of prokaryotic cells is carried out by one or mixtures of agar, agarose, alginate, polyethylene glycol ("PEG"), poly-L-lysine, and cell adhesion molecule ("CAM") ligands. Examples of the CAM ligands include arg-gly-asp ("RGD") peptide, fibronectin, chondronectin, laminin, and the like.

Described below are details of the immobilizing of prokaryotic cells on a surface of an electrode or a transistor with an immobilizing agent. In a case of employing agar, agarose or alginate as an immobilizing agent, the immobilization of prokaryotic cells to an electrode or a transistor is conducted by way of treating the immobilizing agent to be in a sol state (e.g., fluid liquid), dispersing numerous prokaryotic cells in the immobilizing agent in the sol state, attaching the immobilizing agent in which the prokaryotic cells are dispersed onto the surface of an electrode or a transistor by a common spotting method, and lastly, treating the immobilizing agent to be in a gel state.

Further, in a case of using PEG, poly-L-lysine or CAM ligands as an immobilizing agent, the prokaryotic cells are immobilized on the surface of an electrode or a transistor by coating the immobilizing agent thereon and then disposing the prokaryotic cells on the coated immobilizing agent by a common disposing method (e.g., spraying method).

The prokaryotic cells used in the present invention are preferably bacteria, and the toxic substances applicable thereto are preferably substances capable of directly or indirectly inhibiting oxidative phosphorylation of the cell membrane of the prokaryotic cells.

The term "electric signal" used herein means at least one signal of an electric current, electric potential, impedance and capacitance. In exemplary embodiments, the electric signal is impedance. Further, the impedance is preferably dielectric permittivity (E') or dielectric losses (E"). The method of the present invention assesses an adverse effect of a toxic substance from a change in electric signal values measured before and after treatment of the toxic substance. For example, the adverse effect of a toxic substance can be determined by measuring a difference between an initial impedance value measured after immobilization of cells and an impedance value after treatment of the immobilized cells with the toxic substance. Namely, the change in an electric signal in E' is determined based on a difference between patterns exhibited in a specific frequency band. The change in E" is determined based on a difference between positive inflection points.

Further, in addition to the exemplary method described above, an exemplary embodiment of the present invention provides an apparatus for electrically detecting an adverse effect of toxic substances. The apparatus of the present invention includes a measuring part capable of measuring a change in an electric signal of cell membranes of prokaryotic cells and the prokaryotic cells immobilized on the surface of the measuring part.

The measuring part used in the apparatus of the present invention is preferably an electrode or a transistor. The electrode preferably has an interdigital electrode structure, and the transistor is preferably a field effect transistor ("FET") or a thin film transistor ("TFT").

The immobilization, prokaryotic cells, toxic substances, and electric signals employed in the apparatus of the present invention have the same meaning as described in the above exemplary method, and therefore, their descriptions will be omitted here for brevity.

The method and apparatus for electrically detecting an adverse effect of toxic substances according to the present invention are capable of rapidly detecting a whole change as an electric signal within cells caused by the presence of toxic substances. Further, the method and apparatus of the present invention can detect the presence of an adverse effect regardless of the type of toxic substance present. Accordingly, the method and apparatus of the present invention can be easily applied to a lab-on-α-chip and shows high applicability to various fields.

The present invention will now be described in more detail with reference to the following examples. It should be noted that these examples illustrate the present invention merely and thus should not be interpreted to limit the scope of the present invention.

### Example 1

After a suspension of *E. coli* BL21 cells (1×10¹⁰ cells) was mixed with 1 mℓ of 1.5% agarose, 1 µ*ℓ* of the resulting mixture was laid and immobilized on the surface of a positive dielectrophoresis ("DEP") chip. Herein, the positive DEP chip had an interdigit electric structure in which an electrode gap is 15 µm, an electrode width is 15 µm, and an electrode pitch is 15 µm.

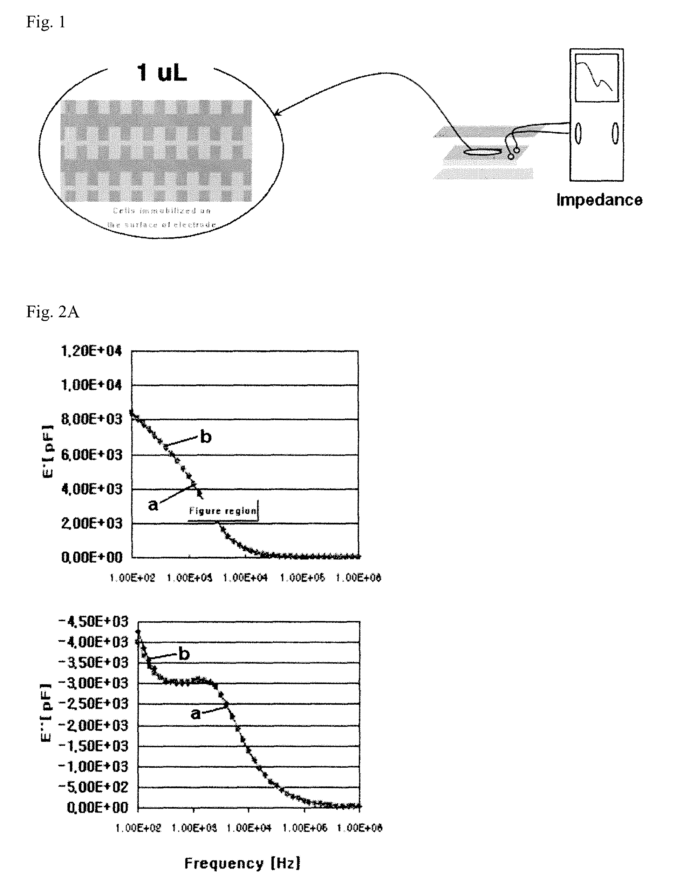

Subsequently, the cells-immobilized chip was soaked in 10 mℓ of 1 µM KCl solution and its initial dielectric permittivity (E') and dielectric losses (E") were measured. After that, 20% ethanol, which causes damage to proteins and cell membranes, was dissolved in 10 mℓ of 1 µM KCl solution, and the cells-immobilized chip was soaked therein for 5 minutes. The chip was then taken out of the 10 mℓ of 1 µM KCl solution and washed with 1 µM KCl solution three times. After soaking the chip in 10 mℓ of 1 µM KCl solution, dielectric permittivity (E') and dielectric losses (E") were measured. FIG 1 shows an apparatus for measuring impedance by using the immobilized cells on the surface of an electrode.

Control 1 is a case of treating only 20% ethanol as a toxic substance to the chip immobilized with only agarose (no cells), which shows the effect (noise) of the toxic substance on agarose. Control 2 is a case of treating only 1 µM KCl solution to the chip immobilized with *E. coli* cells, which represents the noise of prokaryotic cells themselves that varies with the passage of time. The experiments for Controls 1 and 2 were conducted according to a method similar to the above-described method in which the cells-immobilized chip was treated with 20% ethanol as a toxic substance.

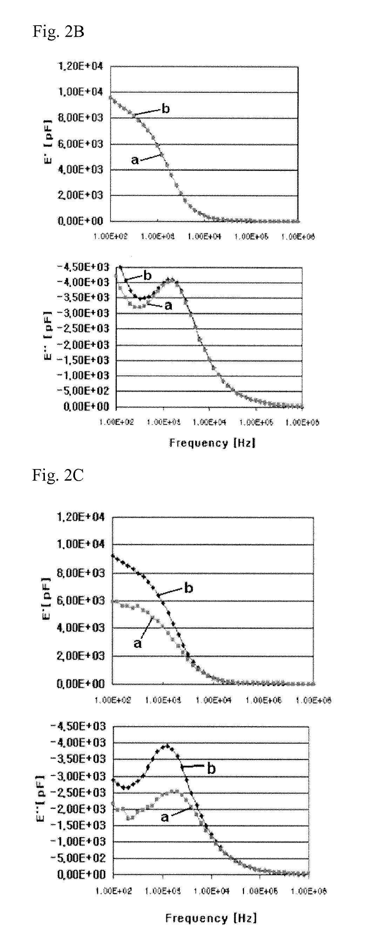

The results of measuring impedance are illustrated in FIGS. 2A to 2C. FIG 2A is the result of Control 1, FIG 2B the result of Control 2 and FIG 2C the result of treating the immobilized cells with a toxic substance. In FIGS. 2A to 2C, Line b represents an initial impedance value measured after the cell immobilization, and Line a represents an impedance value measured after treating with a toxic substance or 1 µM KCI solution. For reference, the criteria for evaluating a signal change in E' is a difference between patterns exhibited at a frequency ranging from 1.00E+02 to 1.00E+04, and the criteria therefor in E" is a difference between positive inflection points.

As can be seen from FIGS. 2A to 2C, while no change was in E' and E" signals of both Controls 1 and 2 measured before and after treatment of a toxic substance or 1 µM KCl solution, the E' and E" signals measured after treating the immobilized cells with a toxic substance decreased compared with the initial impedance value. From these results, it was confirmed that when the prokaryotic cells are treated with a toxic substance, their impedance signals E' and E" are considerably changed. However, in a case of employing other prokaryotic cells other than *E. coli* cells, the E' and E" signals after treatment of a toxic substance may increase compared with the initial impedance value.

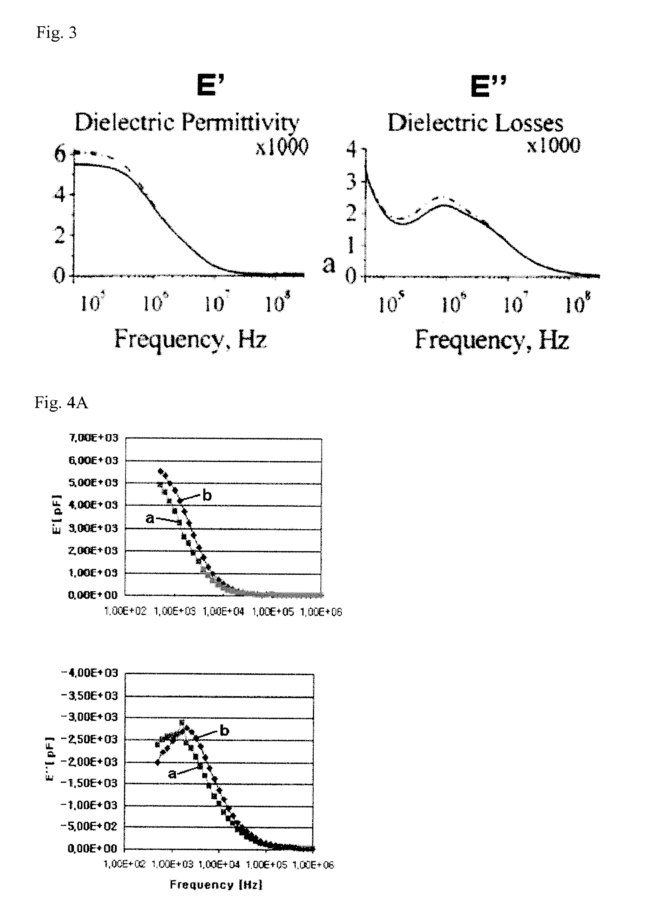

Meanwhile, it was indirectly found from an article entitled "Analysis of Dielectric Spectra of Eukaryotic Cells by Computer Modeling, " Eur. Biophysics J, Vol. 29, Fig. 1 of pp, 141-149, that the aforesaid changes in the E' and E" signals are due to the change of the cell membrane. FIG 3 shows a change in E' (dielectric permittivity) and E" (dielectric losses) signal patterns of the cell membrane that is disclosed in the above article.

### Example 2

In order to examine whether the prokaryotic cells are sensitive only to a toxic substance, 20% sorbitol and 1 × Luria Bertani ("LB") broth were used as a non-toxic substance, and 5 µM carbonyl cyanide m-chloropheoxyhydrazone ("CCCP"), which is known to inhibit an electron transfer of a respiratory system, was used as a toxic substance. The experiment was conducted according to the same method as described in Example 1, except for using the above non-toxic substance and CCCP as the toxic substance instead of ethanol.

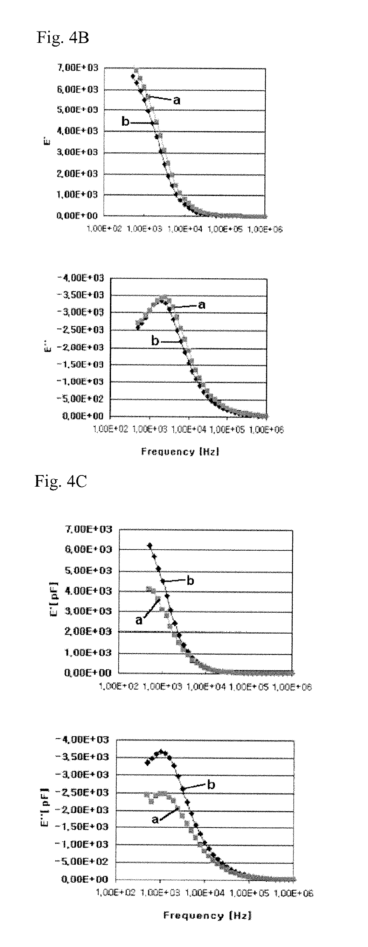

The results are shown in FIGS. 4A to 4C, wherein FIG. 4A is the result of treating with 20% sorbitol, FIG 4B the result of treating with LB broth and FIG 4C the result of treating with CCCP as a toxic substance. In FIGS. 4A to 4C, Line b represents an initial impedance value measured after the cell immobilization, and Line a denotes an impedance value measured after treating with a toxic substance or a non-toxic substance.

As illustrated in FIGS. 4A to 4C, when comparing the electric signals measured before and after treatment with a toxic substance with those measured before and after treatment with a non-toxic substance, it was found that while no meaningful change was in E' and E" signals in the case of treating with a non-toxic substance, the significant reduction occurred in the E' and E" signals over the initial impedance value in the case of treating with CCCP as a toxic substance. Accordingly, it was confirmed that the prokaryotic cells sensitively react to only a toxic substance, which leads to a change in the impedance signals E' and E".

### Example 3

To verify the superiority of the electric signal-based detection method of the present invention, the exemplary method was compared with the existing colony counting and optical detection methods. The optical detection method was performed using a Baclight(TM) Bacterial Membrane Potential Kit (commercially available from by Molecular Probes Inc., U.S.A.) for verification.

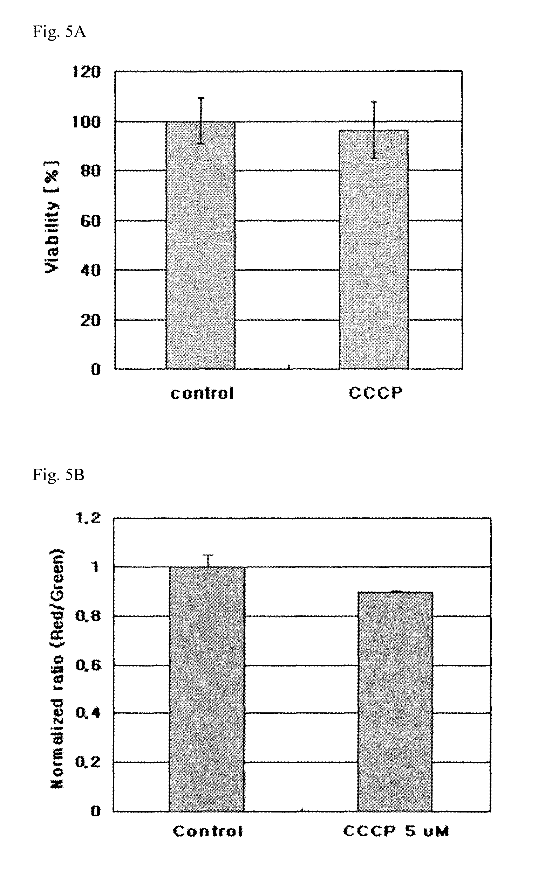

FIG 5A shows a bar graph showing the results of colony counting when the cells-immobilized chip is treated with 5 µM CCCP and a case of an untreated control. As can be seen from FIG 5A, it was difficult to measure an adverse effect of a toxic substance via the colony counting method.

FIG 5B shows a bar graph showing the results of optically measuring a change in cell membrane potentials when the cells-immobilized chip is treated with 5 µM CCCP and a case of an untreated control. As shown in FIG 5B, it was found that since the reduction of cell membrane potential caused by CCCP is only 10% and does not show any significant change compared with the control, the optical detection method is also unsuited to detect an adverse effect of a toxic substance.

However, the method of the present invention showed that the impedance signal (E") of the cell membrane measured after treating with 5 µM CCCP was reduced by about 32% compared with the untreated control (see FIG 4C). From these results, it was confirmed that the adverse effect detecting method of the present invention is much superior to the existing methods of the prior art.

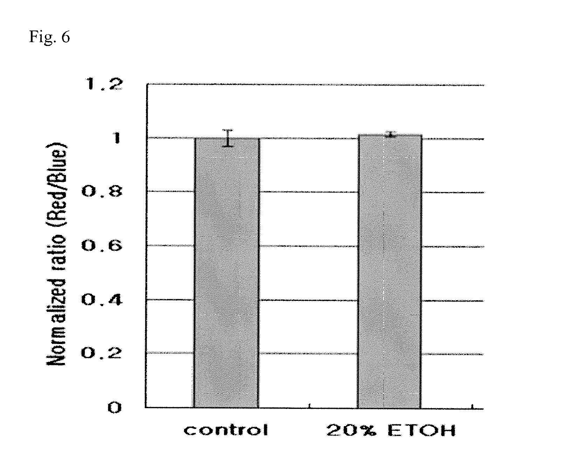

In a case of employing 20% ethanol instead of CCCP, it was also found that while it was difficult to measure an adverse effect of a toxic substance using the colony counting method and the reduction in the cell membrane potential measured by the optical method was almost nothing (see FIG 6), the method of the present invention showed that the reduction in the cell membrane's impedance (E') signal is about 36%. From these results, it can be seen that the method of the present invention is more suitable for detecting an adverse effect of a toxic substance compared with the existing methods of the prior art (see FIG 2C).

As described above, the method and apparatus of the present invention can rapidly detect a whole intracellular change caused by the presence of a toxic substance as an electric signal and also measure an adverse effect regardless of the type of a toxic substance. Accordingly, the method and apparatus of the present invention can be easily and efficiently applied to various fields including a lap-on-α-chip.

While the present invention has been shown and described with respect to particular exemplary embodiments, it will be apparent to those skilled in the art that many changes and modifications may be made without departing from the spirit and scope of the invention as defined in the appended claims.

## Claims

1. A method for detecting an adverse effect of a toxic substance on prokaryotic cells, the method comprising the steps of:
immobilizing the prokaryotic cells on a surface of an electrode or a transistor;
contacting the immobilized prokaryotic cells with a sample containing the toxic substance to be analyzed; and
measuring an electric signal of cell membranes of the prokaryotic cells.

2. The method according to claim 1, wherein the immobilizing the prokaryotic cells is carried out by using at least one immobilizing agent selected from a group of agar, agarose, alginate, polyethylene glycol (PEG), poly-L-lysine and cell adhesion molecule (CAM) ligands.

3. The method according to claim 1, wherein the toxic substance is a substance that directly or indirectly inhibits an oxidative phosphorylation of the cell membranes of the prokaryotic cells.

4. The method according to claim 1, wherein the electric signal is at least one signal selected from a group of electric current, electric potential, impedance and capacitance.

5. The method according to claim 4, wherein the impedance is one of dielectric permittivity (E') and dielectric losses (E").

6. An apparatus for detecting an adverse effect of a toxic substance using prokaryotic cells, the apparatus comprising:
a measuring part which detects a change in an electric signal of cell membranes of the prokaryotic cells; and
the prokaryotic cells immobilized on the measuring part.

7. The apparatus according to claim 6, wherein the measuring part is one of an electrode and a transistor.

8. The apparatus according to claim 7, wherein the electrode has an interdigital electrode structure.

9. The apparatus according to claim 6, wherein the immobilization is carried out by using at least one immobilizing agent selected from a group of agar, agarose, alginate, polyethylene glycol (PEG), poly-L-lysine and cell adhesion molecule (CAM) ligands.

10. The apparatus according to claim 6, wherein the electric signal is at least one signal selected from a group of electric current, electric potential, impedance and capacitance.

11. The apparatus according to claim 10, wherein the impedance is one of dielectric permittivity (E') and dielectric losses (E").
